# EUROPÄISCHE PATENTSCHRIFT

(11) **EP 1 773 223 B1**
(45) Veröffentlichungstag und Bekanntmachung des Hinweises auf die Patenterteilung: **23.03.2011**
(21) Anmeldenummer: 05751795.5
(22) Anmeldetag: 19.05.2005
(51) Int. Cl.: A61B 18/00

(54) **EINRICHTUNG FÜR DIE ARGON-PLASMA-KOAGULATION**
DEVICE FOR ARGON PLASMA COAGULATION
DISPOSITIF DE COAGULATION PAR PLASMA D`ARGON

(30) Priorität: 11.06.2004 DE 102004028362; 14.07.2004 DE 102004033975
(43) Veröffentlichungstag der Anmeldung: 18.04.2007
(73) Patentinhaber: Erbe Elektromedizin GmbH, 72072 Tübingen (DE)
(72) Erfinder: KÜHNER, Ralf, 70567 Stuttgart (DE)
(74) Vertreter: Bohnenberger, Johannes
(86) Internationale Anmeldenummer: PCT/EP2005/005454
(87) Internationale Veröffentlichungsnummer: WO 2005/120372

(56) Entgegenhaltungen:
- FR-A- 2 657 489
- US-A1- 2001 018 587
- US-A1- 2003 105 458
- US-B1- 6 602 249

## Beschreibung

Die Erfindung betrifft eine Einrichtung für die Argon-Plasma-Koagulation (APC) nach dem Oberbegriff des Patentanspruches 1.

Die Hochfrequenzchirurgie, der auch die Argon-Plasma-Koagulation als Teilgebiet zuzuordnen ist, wird seit vielen Jahren sowohl in der Human- als auch in der Veterinärmedizin eingesetzt, um biologisches Gewebe zu koagulieren und/oder zu schneiden. Dabei wird mit Hilfe geeigneter elektrochirurgtscher Instrumente hochfrequenter Strom durch das zu behandelnde Gewebe geleitet, so dass sich dieses aufgrund Eiweißkoagulation und Dehydratation verändert. Es lassen sich somit aufgrund eines Koagulationsprozesses Gefäße verschließen und Blutungen stillen. Ein auf den Koagulationsprozess folgender Schneidprozess ermöglicht dann die vollständige Trennung bereits koagulierten Gewebes.

Die Argon-Plasma-Koagulation ermöglicht ein berührungsfreies Koagulieren von Gewebe und dient der effektiven Blutstillung und Gewebedevitalisierung. Bei dieser Koagulationsart wird inertes Arbeitsgas, z. B. Argon, über Gaszuführungseinrichtungen von einem Argon-Plasma-Koagulations-Instrument zur Argon-Dosierung und Fehlerüberwachung an das zu behandelnde Gewebe geleitet. Mit Hilfe des Arbeitsgases kann dann ein PlasmaStrahl zwischen einem distalen Ende der Gaszuführungseinrichtungen, beispielsweise einer Sonde, und dem Gewebe erzeugt werden. Der HF-Strom lässt sich dann an dem zu behandelnden Gewebe applizieren, ohne dass das elektrochirurgisches Instrument mit dem Gewebe in Kontakt kommt. Ein Verkleben des Gewebes mit den Instrumenten wird somit vermieden. Die Argon-Plasma-Koagulation verhindert zudem die Karbonisierung des Gewebes sowie Rauchbildung und Geruchsbelästigung.

Die Technik der Argon-Plasma-Koagulation kann sowohl am eröffneten Körper als auch minimal-invasiv durchgeführt werden. In letztgenannter Anwendung wird die Sonde zum Zuführen des Arbeitsgases beispielsweise durch ein Endoskop auf dem Weg einer natürlichen Körperöffnung zum Operationsherd in die betreffende Körperhöhle vorgeschoben. Ein Nachteil bekannter APC-Einrichtungen und deren Sonden besteht darin, dass beim Einführen der Sonde in die Körperhöhle oder bei schon eingeführter Sonde von einem distalen Ende der Sonde Arbeitsgas, körpereigene Gase und/oder körpereigene Flüssigkeiten in die Gaszuführungseinrichtungen zurückfließen können. Dabei werden die Sonde selbst, aber auch mit dieser verbundene weitere Elemente der Einrichtung zur Argon-Plasma-Koagulation, wie z. B. das APC-Instrument, verschmutzt.

Einige bekannten Vorrichtungen sehen daher sterile, hydrophobe Membranfilter vor, die vorzugsweise an einem distalen Ende der Gaszuführungseinrichtungen, d. h. insbesondere in der Sonde, platziert werden und so einen Rückfluss von Gasen und/oder Flüssigkeiten verhindern sollen. Solche Filter sind jedoch teuer, müssen regelmäßig gewechselt werden und verursachen daher einen enormen Wartungsaufwand und hohe Kosten. Darüber hinaus besteht die Gefahr, den Filterwechsel zu vergessen und eine nachfolgende Operation mit einem kontaminierten Gerät durchzuführen. Einmal kontaminierte Geräte sind im Übrigen nicht mehr zuverlässig aufzubereiten.

Alternativ ist bei den bekannten Geräten vorgesehen, kontinuierlich Arbeitsgas über die Sonde an das zu behandelnde Gewebe fließen zu lassen. Damit wird ebenfalls ein retrograder Fluss vermieden. Ein andauernder Hygienefluss flutet allerdings die betroffene Körperhöhle unnötig mit dem inerten Arbeitsgas, was leicht zu Komplikationen während der Operation führen kann. Zudem muss die gesamte APC-Einrichtung bereits beim Einbringen der Sonde in Betrieb sein, um den Gasfluss zu gewährleisten. Auch müssen Gerät und Gasfluss so lange eingeschaltet bleiben, bis die Sonde vollständig aus der Körperhöhle entfernt ist.

Näheres zum Stand der Technik kann den Druckschriften FR 2 657 489 A, US 2003/105458 A1, US 6 602 249 B1 und US 2001/018587 A1 entnommen werden. Jedes dieser Dokumente offenbart eine Einrichtung für die Argon-Plasma-Koagulation mit Gaszuführungseinrichtungen, mittels welcher Arbeitsgas in einer Strömungsrichtung an ein zu behandelndes Gewebe geführt werden kann, wobei dabei das Arbeitsgas am distalen Ende der Gaszuführungseinrichtungen ausströmt, und mit an die Gaszuführungseinrichtungen angeschlossene Sperreinrichtungen, die geeignet sind, eine Gasströmung in den Gaszuführungseinrichtungen entgegen der Strömungsrichtung zu sperren.

Der Erfindung liegt die Aufgabe zugrunde, eine Einrichtung für die Argon-Plasma-Koagulation der eingangs genannten Art dahin gehend weiter zu bilden, dass eine Kontaminierung der Gaszuführungseinrichtungen und/oder des Argon-Plasma-Koagulations-Instrumentes mit Arbeitsgas, körpereigenen Gasen und/oder körpereigenen Flüssigkeiten verhindert wird, und dass der Gasweg für das Arbeitsgas ohne großen Aufwand in strömungsrichtung freigegelen werden bann.

Diese Aufgabe wird durch eine Einrichtung für die Argon-Plasma-Koagulation nach Patentanspruch 1 gelöst.

Die Gaszuführungseinrichtungen sind derart ausgebildet, dass ein retrograder Fluss von Arbeitsgas, körpereigenen Gasen und/oder körpereigenen Flüssigkeiten in die Gaszuführungseinrichtungen unterbunden wird. Die Gaszuführungseinrichtungen weisen dazu den Rückfluss verhindernde Elemente auf, die weder des Austauschens noch einer gesonderten Reinigung bedürfen.

Ein wesentlicher Punkt der Erfindung liegt darin, dass die Sperreinrichtung durch eine Betätigungsvorrichtung öffenbar ist, wobei die Betätigungsvorrichtung an einem Element der Gaszuführungseinrichtungen angeschlossen oder an dieses anschließbar ist, das in einer Richtung entgegen der Strömungsrichtung und damit vor einem die Sperreinrichtung aufweisenden Element angeordnet ist, so dass ein Öffnen der Sperreinrichtung durch ein Verbinden beider Elemente erfolgt.

Bevorzugte Weiterbildungen der Erfindung sind in den Unteransprüchen angegeben.

In einer ersten bevorzugten Ausführungsform ist die Sperreinrichtung an einem Rohr, einer schlauchförmigen Sonde oder dergleichen chirurgischen Instrument der Gaszuführungseinrichtungen angeschlossen oder an dieses anschließbar. Insbesondere ist hier die Anordnung der Sperreinrichtung an einem distalen Ende des chirurgischen Instruments, beispielsweise der Sonde, vorteilhaft, weil jeglicher Gas- und/oder Flüssigkeitsrückfluss aus dem Operationsgebiet frühzeitig verhindert wird. Es können nur sehr wenige kontaminierende Gase und/oder Flüssigkeiten in die Sonde eindringen, weil sich ein zur Verfügung stehendes Volumen nur vom distalen Ende der Sonde bis zu der unmittelbar danach platzierten Sperreinrichtung erstreckt. Ein restliches Lumen der Sonde und auch weitere an die Sonde angeschlossene Elemente der Gaszuführungseinrichtungen bleiben sauber, so dass deren Reinigung und Sterilisation erheblich erleichtert wird.

Das Anordnen der Sperreinrichtung an dem distalen Ende der Sonde hat zudem den Vorteil, dass in der Sonde vorhandenes Gas, insbesondere Arbeitsgas, auch in nicht-aktiviertem Zustand der APC-Einrichtung nicht ausströmen kann. Dadurch ist die Sonde,
sofern einmal mit Arbeitsgas gefüllt, schneller betriebsbereit, wodurch die Anwendungssicherheit erhöht wird.

In einer weiteren bevorzugten Ausführungsform ist die Sperreinrichtung an einem Anschlussstück des chirurgischen Instruments der Gaszuführungseinrichtungen angeschlossen oder an dieses anschließbar. Chirurgische Instrumente, wie z. B. Sonden, weisen oftmals explizite Anschlussstücke, also beispielsweise explizite Sondenstecker auf. Ist die Sperreinrichtung in dem Anschlussstück vorgesehen, so können insbesondere weitere, an dem Anschlussstück angeschlossene Elemente der Gaszuführungseinrichtungen zuverlässig vor Verunreinigung geschützt werden.

In einer bevorzugten Ausführungsform ist die Sperreinrichtung an Anschlussleitungen zum Anschließen des chirurgischen Instruments an ein Argon-Plasma-Koagulations-Instrument der Einrichtung für die Argon-Plasma-Koagulation angeschlossen oder an diese anschließbar. Vorzugsweise ist die Anordnung in den Anschlussleitungen als zusätzliche Maßnahmen, d. h. als zusätzliche Sperreinrichtung vorgesehen, um neben dem Schutz der Anschlussleitungen insbesondere den Schutz des APC-Instrumentes vor eindringendem Arbeitsgas, eindringenden körpereigenen Gasen und/oder körpereigenen Flüssigkeiten weiter zu erhöhen.

Vorteilhafterweise ist die Sperreinrichtung an einem an dem Argon-Plasma-Koagulations-Instrument angeordneten Gasanschlussstück angeschlossen oder an dieses anschließbar. Damit wird insbesondere das APC-Instrument zuverlässig vor retrograd einlaufenden Gasen bzw. einströmenden Flüssigkeiten geschützt. Die Anordnung der Sperreinrichtung unmittelbar an dem APC-Instrument ist insbesondere dann vorzusehen, wenn die Anschlussleitungen und/oder das chirurgische Instrument, also beispielsweise die Sonde, als Einmal-Artikel vorgesehen sind, so dass sich hier der Einbau der Sperreinrichtung als zu kostspielig erweisen würde. Allerdings ist die an dem Gasanschlussstück vorgesehene Sperreinrichtung auch dafür geeignet, insbesondere das APC-Instrument gesondert und zuverlässig zu schützen, wobei auch die Sonde und die Anschlussleitungen eine entsprechende Sperreinrichtung aufweisen können.

Eine erfindungsgemäße Lösung sieht vor, die Sperreinrichtung als eine mechanisch und/oder elektrisch betätigbare Einrichtung auszubilden. Mechanisch und/oder elektrisch betätigbare Sperreinrichtungen sind handelsübliche, kostengünstige Bauelemente, wie z. B. Ventile, die an die gewünschte Stelle einfach angeschlossen werden können. Die mechanisch und/oder elektrisch betätigbare Einrichtung ermöglicht dann auf einfachste Weise die Steuerung einer Durchflussrichtung und einer Gasmenge zu einem beliebigen Zeitpunkt.

Vorteilhafterweise ist die Sperreinrichtung als Sperrventil, beispielsweise als Rückschlagventil, ausgebildet. Rückschlagventile sind einfache und kostengünstige Bauelemente, die einen retrograden Fluss von Arbeitsgas, körpereigenen Gasen und/oder körpereigenen Flüssigkeiten zuverlässig verhindern, Ein Ventil dieser Art ist zudem für den Fall, dass kein chirurgisches Instrument, beispielsweise die Sonde, an der APC-Einrichtung angeschlossen ist, in beiden Richtungen abdichtend. So kann z. B. ein Austritt von Restgas aus den Anschlussleitungen vermieden werden, auch wenn deren die Sonde aufnehmendes Ende offen liegt. Rückschlagventile eignen sich insbesondere zum Einbau in solche Elemente der Einrichtung für die Argon-Plasma-Koagulation, die wiederaufbereitet und demgemäß mehrmals eingesetzt werden sollen. Rückschlagventile können Kegel-, Kugel- oder auch Tellerelemente aufweisen, wobei das jeweilige Element beispielsweise entgegen einer Federkraft durch einen von einem Gas oder einer Flüssigkeit bewirkten Druck öffnet. Bei einer Beaufschlagung des Elements in Sperrrichtung wirkt zusätzlich zu der Federkraft die durch den Druck bewirkte Kraft auf den Ventilsitz, so dass das Ventil sperrt. Rückschlagventile oder dergleichen Sperrventile lassen sich jedoch auch manuell, beispielsweise über ein Hahnrad oder einen Drehgriff, betätigen.

In einer bevorzugten Ausführungsform ist das Sperrventil als Lippendichtung, d. h. als Entenschnabelventil ausgebildet. Die Lippendichtung ist vorteilhafterweise so ausgestaltet, dass sich die Lippen beim Fluss des Arbeitsgases in Strömungsrichtung öffnen, während sie beim Rückfluss von Arbeitsgas, körpereigenen Gasen und/oder körpereigenen Flüssigkeiten bauartbedingt schließen. Der Strömungswiderstand von Lippendichtungen in Strömungsrichtung ist nicht nennenswert, der Verschluss in Gegenrichtung jedoch zuverlässig. Die Lippendichtung eignet sich insbesondere für das Platzieren im Lumen der Sonde.

Alternativ oder auch zusätzlich kann in den Gaszuführungseinrichtungen mindestens ein Drosselventil angeschlossen sein. Das Drosselventil ist als einstellbarer Widerstand ausgebildet und beeinflusst den Volumenstrom derart, dass ein Durchfluss erst ab einem bestimmten, von einem Gas oder einer Flüssigkeit bewirkten Druck möglich ist. Das Ventil kann dann beispielsweise so eingestellt sein, dass zum Öffnen des Ventils der von dem Arbeitsgas in Strömungsrichtung aufgebrachte Gasdruck erforderlich ist. Ein retrograder Fluss von Arbeitsgas, körpereigenen Gasen und/oder körpereigenen Flüssigkeiten dagegen erreicht den erforderlichen Druck aufgrund einer zu geringen Strömungsgeschwindigkeit nicht, so dass das Ventil entgegen der Strömungsrichtung sperrt. Die Strömungsrichtung ist hier definiert als Richtung des Gasflusses von dem APC-Instrument zu dem Operationsgebiet. Ein Drosselrückschlagventil würde einen retrograden Fluss auch unabhängig vom Druck grundsätzlich verhindern.

Oftmals wird das chirurgische Instrument, also z. B. die Sonde, über einen Arbeitskanal eines Endoskops an das Operationsgebiet herangeführt, ohne dass das Instrument mit dem APC-Instrument über die Anschlusseinrichtungen verbunden ist. Aufgrund einer Druckdifferenz zwischen Körperhöhle und Gaszuführungseinrichtungen können Arbeitsgas, körpereigene Gase und/oder körpereigene Flüssigkeiten in die Sonde eindringen. Um dies zu vermeiden, ist in dem Instrument die den Rückfluss verhindernde und durch eine explizite Betätigungsvorrichtung öffenbare Sperreinrichtung vorgesehen. Wird das die Sperreinrichtung aufweisende Element mit dem die Betätigungsvorrichtung aufweisenden Element verbunden, so können Sperreinrichtung und Betätigungsvorrichtung derart zusammenwirken, dass die Sperreinrichtung durch die Betätigungsvorrichtung in Strömungsrichtung geöffnet wird. Damit kann der Gasweg für das Arbeitsgas ohne großen Aufwand in Strömungsrichtung freigegeben werden.

Vorzugsweise erstreckt sich die Betätigungsvorrichtung aus dem ihr zugehörigen Element der Gaszuführungseinrichtungen hervor. Eine Verbindung mit dem die Sperreinrichtung aufweisenden Element führt dann zwangsweise zu einem Kontakt von Sperreinrichtung und Betätigungsvorrichtung, so dass die Sperreinrichtung betätigt, d. h. geöffnet wird.

Ein Öffnen erfolgt beispielsweise dadurch, dass die hervorstehende Betätigungsvorrichtung durch das Verbinden der Elemente der Gaszuführungseinrichtungen derart in die Sperreinrichtung eindringt, dass diese durchbohrt und aufgrund der so erzeugten Öffnung der Gasfluss in Strömungsrichtung ermöglicht wird.

Ist die Betätigungsvorrichtung hingegen ausschließlich innerhalb des zugehörigen Elements der Gaszuführungseinrichtungen ausgebildet, ist das die Sperreinrichtung aufweisende Element derart in das die Betätigungsvorrichtung aufweisende Element einzuführen, dass auf diese Art ein Zusammenwirken von Betätigungsvorrichtung und Sperreinrichtung ermöglicht wird.

Alternativ ist es möglich, die Sperreinrichtung nicht zu durchbohren, sondern mit Hilfe der hervorstehenden Betätigungsvorrichtung mindestens Teile der Sperreinrichtung aus einer ersten, die Sperreinrichtung in einem geschlossenen Zustand haltenden Position in eine zweite, die Sperreinrichtung in einem geöffneten Zustand haltenden Position zu verschieben.

In einer bevorzugten Ausführungsform ist die Sperreinrichtung als ein durch die Betätigungsvorrichtung irreversibel öffenbares Element ausgebildet. Das irreversibel öffenbare Element ist vorzugsweise als Membran ausgeführt. Eine Membran ist ein äußerst kostengünstiges Bauteil und daher vorzugsweise für Einmalsonden zu verwenden. Die Membran verhindert bei einem Einführen der Sonde in die Körperhöhle einerseits das Einströmen von körpereigenen Gasen und/oder körpereigenen Flüssigkeiten, so dass diese und auch etwaige Anschlussleitungen nicht verunreinigt werden. Andererseits lässt sich die Membran mit der dafür ausgebildeten Betätigungsvorrichtung leicht durchbohren.

In einer weiteren bevorzugten Ausführungsform ist die Sperreinrichtung als durch die Betätigungsvorrichtung mehrfach öffenbares Element ausgebildet. Als mehrfach öffenbares Element eignet sich hier insbesondere eine Membran mit einer definierten Öffnungsstelle, d. h. mit einer Soll-Öffnungsstelle, die jedoch in nicht-durchbohrtem Zustand für Gase und/oder Flüssigkeiten nahezu undurchlässig ist. Mit dem Durchbohren der Membran an der dafür vorgesehenen Öffnungsstelle weichen der Betätigungsvorrichtung mindestens Teile der Membran elastisch aus, so dass der Fluss des Arbeitsgases ermöglicht wird. Bei Entfernung der Betätigungsvorrichtung nehmen die Teile der Membran wieder ihre ursprüngliche Position ein, d. h., die Membran ist wieder geschlossen. Da sich die ausweichenden Teile der Membran an die Betätigungsvorrichtung anschmiegen, sollte die Betätigungsvorrichtung als ein hervorstehender Hohlkörper ausgebildet sein. Damit ist der Fluss des Arbeitsgases durch den Hohlkörper hindurch gewährleistet.

Die elastische Membran kann auch derart ausgebildet sein, dass das in Strömungsrichtung fließende Arbeitsgas aufgrund seines Gasdrucks das Öffnen der Membran bewirkt, während ein retrograder Fluss von Arbeitsgas, körpereigenen Gasen und/oder körpereigenen Flüssigkeiten den für das Öffnen der Membran erforderlichen Druck nicht aufbringt.

Vorzugsweise ist das mehrfach öffenbare Element als Sperreinrichtung mit einem die Gas- und/oder Flüssigkeitsströmung versperrenden und von der Betätigungsvorrichtung verschiebbaren Körper ausgebildet. Der Körper ist beispielsweise mit einem Federelement verbunden, so dass die Betätigungsvorrichtung den Körper entgegen der Federkraft verschiebt und somit der Gasweg für das Arbeitsgas freigegeben ist. Bei Entfernen der Betätigungsvorrichtung stellt das Federelement den Körper in seine ursprüngliche Ausgangslage zurück, so dass die Sperreinrichtung für den retrograden Fluss von Arbeitsgas, körpereigenen Gasen und/oder körpereigenen Flüssigkeiten wieder undurchlässig ist. Die hier beschriebene Anordnung ist eine äußerst zuverlässige Möglichkeit, den retrograden Fluss praktisch gänzlich zu verhindern. Die Anordnung bietet sich insbesondere für wiederverwendbare Elemente der Gaszuführungseinrichtungen an.

In einer bevorzugten Ausführungsform ist die Betätigungsvorrichtung als Dorn, Kanüle oder Stößel ausgebildet. Ein Stößel bietet sich als kostengünstiges Bauteil insbesondere für die mehrfach öffenbare, mit der Betätigungsvorrichtung zusammenwirkende Sperreinrichtung an, während Kanüle oder Dorn vor allem zum Durchbohren der Membran vorgesehen sind. Bei Anwendung einer Kanüle ist der Fluss des Arbeitsgases zusätzlich durch die Kanüle hindurch gewährleistet. Die als Stößel, Kanüle oder auch Dorn ausgebildete Betätigungsvorrichtung lässt sich auf einfache Weise in dem dafür vorgesehenen Element der Gaszuführungseinrichtungen anbringen und ggf. jederzeit erneuern. Alternativ ist es möglich, die Betätigungsvorrichtung integral mit dem Element der Gaszuführungseinrichtungen auszubilden. Das distale Ende des Elements ist dann z. B. angeschrägt oder stößelartig ausgebildet. Damit wird die Handhabung vereinfacht, gleichzeitig lassen sich die Herstellungskosten senken.

Es ist auch möglich, die Betätigungsvorrichtung selbst betätigbar auszubilden, so dass durch das Verbinden der entsprechenden Elemente der Gaszuführungseinrichtungen beispielsweise ein Mechanismus ausgelöst wird, der die Betätigungsvorrichtung in Richtung der Sperreinrichtung bewegt und so ein Öffnen derselben bewirkt. Der Mechanismus könnte dann z. B. als ein Federmechanismus ausgebildet sein.

In einer weiteren bevorzugten Ausführungsform ist vorgesehen, dass die Sperreinrichtung einen Garantieverschluss umfasst. Dies bietet sich insbesondere bei Einmal-Sonden an, bei welchen eine Kennzeichnung eine intakte Sperreinrichtung garantiert.

Nachfolgend wird die Erfindung anhand von Ausführungsbeispielen beschrieben, die anhand der Abbildungen näher erläutert werden. Hierbei zeigen:
- - Fig. 1: eine schematische Darstellung einer Einrichtung für die Argon-Plasma- Koagulation;
- - Fig. 2: ein perspektivisch dargestelltes distales Ende eines Elements von Gaszufüh- rungseinrichtungen mit einer Sperreinrichtung;
- - Fig. 3a: eine Schnittansicht eines proximalen Endes eines Elements von Gaszuführungseinrichtungen mit einer Sperreinrichtung;
- - Fig. 3b: ein distales Ende eines Elements von Gaszuführungseinrichtungen, ausgebil- det, um mit dem Element gemäß Fig. 3a zusammenzuwirken;
- - Fig. 4a: eine Schnittansicht eines proximalen Endes eines Elements von Gaszuführungseinrichtungen mit einer Sperreinrichtung;
- - Fig. 4b: ein distales Ende eines Elements von Gaszuführungseinrichtungen, ausgebil- det, um mit dem Element gemäß Fig. 4a zusammenzuwirken;
- - Fig. 5: eine Schnittansicht eines distalen Endes eines Elements von Gaszuführungs- einrichtungen mit einer Sperreinrichtung und einer Betätigungsvorrichtung;
- - Fig. 6: ein perspektivisch dargestelltes distales Ende eines Elements von Gaszufüh- rungseinrichtungen mit einer Sperreinrichtung und einer Betätigungsvor- richtung;
- - Fig. 7a: eine perspektivische Schnittansicht einer Sperreinrichtung in einem Element einer Gaszuführungseinrichtung;
- - Fig. 7b: eine perspektivische Ansicht der Sperreinrichtung gemäß Fig. 7a in betätigtem Zustand.

In der nachfolgenden Beschreibung werden für gleiche und gleich wirkende Teile dieselben Bezugsziffern verwendet.

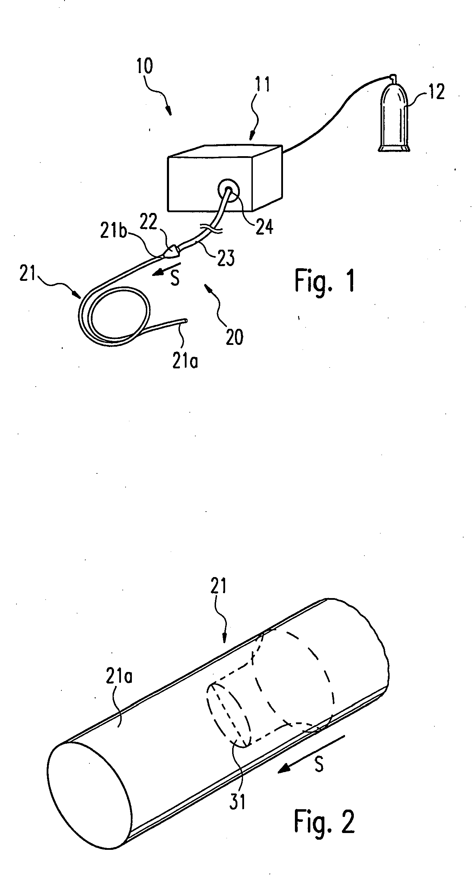

Fig. 1 zeigt eine schematische Darstellung einer Einrichtung für die Argon-Plasma-Koagulation 10 in perspektivischer Ansicht. Dabei ist eine Gasquelle 12 mit einem Argon-Plasma-Koagulations-Instrument 11 verbunden. Die Gasquelle 12 liefert für die Argon-Plasma-Koagulation notweniges Argon oder dergleichen inertes Arbeitsgas. Das APC-Instrument 11 zur Gasdosierung und Fehlerüberwachung weist ein Gasanschlussstück 24 zum Anschließen eines ersten Endes einer Anschlussleitung 23 an das APC-Instrument 11 auf. Ein zweites Ende der Anschlussleitung 23 ist mit einem chirurgischen Instrument, hier einer Sonde 21, über einen Sondenstecker 22 verbunden. Alternativ ist es möglich, die Sonde 21 unmittelbar über den Sondenstecker 22 an dem APC-Instrument 11 anzuschließen. Ferner ist es möglich, die Sonde 21 mit der Anschlussleitung 23 über eine Schraubverbindung zu verbinden. Das Gasanschlussstück 24, die Anschlussleitung 23, der Sondenstecker 22 und die Sonde 21 bilden Gaszuführungseinrichtungen 20 der APC-Einrichtung 10 aus. Die Sonde 21 weist eine Elektrode (nicht gezeigt) zum Zuführen eines hochfrequenten Stromes an das Operationsgebiet auf, wobei der Strom von einem HF-Generator (nicht gezeigt) geliefert wird. Es ist möglich, den HF-Generator mit dem APC-Instrument 11 integral auszubilden.

Die Argon Plasma Koagulation lässt sich sowohl am eröffneten Körper als auch minimal-invasiv durchführen. Bei einem minimal-invasiven Eingriff wird üblicherweise die in der Fig. 1 gezeigte Sonde 21 durch ein Endoskop (nicht gezeigt) auf dem Weg einer natürlichen Körperöffnung zum Operationsgebiet in eine betreffende Körperhöhle vorgeschoben. Aus einem distalen Ende 21a der Sonde 21 strömt Arbeitsgas an den Operationsherd, so dass zwischen der Sonde 21 und einem zu behandelnden Gewebe ein Argon-Plasma-Strahl erzeugt wird. Aufgrund des Plasmas kann der Strom berührungslos über dieses, also über das ionisierte Arbeitsgas an dem Gewebe appliziert werden.

Um einen Rückfluss von Arbeitsgas, körpereigenen Gasen und/oder körpereigenen Flüssigkeiten wie Blut oder sonstige Gewebeflüssigkeiten in die Gaszuführungseinrichtungen 20 zu vermeiden, weisen diese gemäß vorliegender Erfindung mindestens eine Sperreinrichtung auf (in Fig. 1 nicht gezeigt), um diesen Rückfluss zu unterbinden. Die Sperreinrichtung ist dazu mindestens in einem Element der Gaszuführungseinrichtungen 20 angeschlossen oder an dieses anschließbar. So ist die Sperreinrichtung mindestens an dem chirurgischen Instrument, also z. B. an der Sonde 21, vorgesehen, um den Rückfluss möglichst frühzeitig zu unterbinden. Weisen die Anschlussleitungen 23 und/oder der Sondenstecker 22 eine Sperreinrichtung auf, so werden die Anschlussleitungen zusätzlich vor einer Kontaminierung geschützt. Insbesondere sollte die Sperreinrichtung aber auch in oder an dem Gasanschlussstück 24 des APC-Instrumentes 11 angeordnet sein, um gerade hier eine Verunreinigung zu vermeiden.

Fig. 2 zeigt ein perspektivisch dargestelltes Element von Gaszuführungseinrichtungen 20, beispielsweise ein distales Ende 21a einer Sonde 21 mit einer Sperreinrichtung. Die Sperreinrichtung ist hier als Lippendichtung 31 ausgeführt. Lippendichtungen werden auch als Entenschnabelventil bezeichnet. Die Lippendichtung 31 lässt sich in einer gewünschten Strömungsrichtung S des Arbeitsgases, nämlich in Richtung von dem APC-Instrument 11 hin zum Operationsgebiet öffnen, während sie beim Rückfluss von Arbeitsgas, körpereigenen Gasen und/oder körpereigenen Flüssigkeiten bauartbedingt schließt.

An Stelle der Lippendichtung 31 lässt sich jede gewünschte Art von Ventilen, insbesondere von Sperrventilen, in ein Element der Gaszuführungseinrichtungen einbauen. Diese verhindern zuverlässig den retrograden Fluss, so dass eine Kontaminierung der Gaszuführungseinrichtungen 20 vermieden-wird. Auch Drossel oder-Drosseltüekschlagventile sind vorzugsweise in mehrfach verwendbaren Gaszuführungseinrichtungen 20 als zuverlässige Rückflusssperren einbaubar. Das Drosselventil ist als einstellbarer Widerstand ausgebildet und beeinflusst den Volumenstrom derart, dass ein Durchfluss in Strömungsrichtung erst ab einem bestimmten, von einem Gas oder einer Flüssigkeit bewirkten Druck möglich ist. Das Ventil kann dann beispielsweise so eingestellt sein, dass zum Öffnen des Ventils der von dem Arbeitsgas in Strömungsrichtung aufgebrachte Gasdruck erforderlich ist. Ein retrograder Fluss von Arbeitsgas, körpereigenen Gasen und/oder körpereigenen Flüssigkeiten dagegen erreicht den erforderlichen Druck aufgrund einer zu geringen Strömungsgeschwindigkeit nicht, so dass das Ventil entgegen der Strömungsrichtung sperrt.

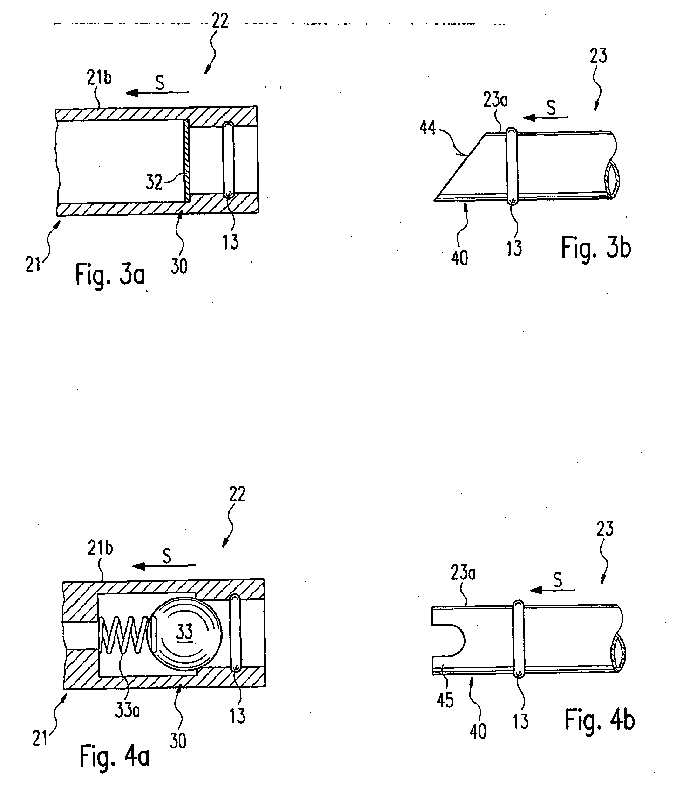

Fig. 3a zeigt eine Schnittansicht eines Elements von Gaszuführungseinrichtungen 20, beispielsweise eines proximalen Endes 21b einer Sonde 21 mit einer Sperreinrichtung 30. Das proximale Ende 21b umfasst in der praktischen Anwendung ein Anschlussstück, d. h. in diesem Falle einen Sondenstecker 22. Dieser ist gemäß Fig. 3a mit einer Membran 32 als Sperreinrichtung ausgebildet. Fig. 3b zeigt eine als distales Ende eines Elements von Gaszuführungseinrichtungen 20 ausgebildete Betätigungsvorrichtung 40, z. B. ein distales Ende 23a einer Anschlussleitung 23 zum Verbinden der Sonde 21 mit dem APC-Instrument 11, wobei das Element ausgebildet ist, um mit dem Element gemäß Fig. 3a zusammenzuwirken. Die Betätigungsvorrichtung 40 ist mit einer Anschrägung 44 ausgebildet und derart ausgelegt, das sie in das proximale Ende 21b der Sonde 21 bzw. in den Sondenstecker 22 einführbar ist. Sobald Sonde 21 und Anschlussleitung 23 verbunden werden, durchbohrt das distale Ende 23a der Anschlussleitung 23 die Membran 32 aufgrund der Anschrägung 44 und gibt einen Gasweg für ein Arbeitsgas in Strömungsrichtung S von dem APC-Instrument 11 hin zu dem Operationsgebiet frei.

Alternativ ist es möglich, dass Fig. 3b eine Kanüle zeigt, die beispielsweise an oder in einer Anschlussleitung explizit vorgesehen ist und die entsprechend obiger Beschreibung wirkt.

Das Zusammenwirken von zwei miteinander verbindbaren Elementen der Gaszuführungseinrichtungen 20, d. h. das Zusammenwirken der Sperreinrichtung 30 mit der Betätigungsvorrichtung 40 zum Öffnen der Sperreinrichtung 30 stellt eine äußerst einfache und kostengünstige Möglichkeit dar, den Weg nur in gewünschter Strömungsrichtung S freizugeben. Dies ist insbesondere dann vorteilhaft, wenn das chirurgische Instrument, also z. B. die Sonde 21, in die entsprechende Körperhöhle eingeführt werden soll, ohne mit dem APC-Instrument 11 verbunden zu sein. Aufgrund einer Druckdifferenz zwischen Körperhöhle und Gaszuführungseinrichtungen können Gase und/oder Flüssigkeiten leicht in die Sonde 21 eindringen. Die Sperreinrichtung 30 ist deshalb derart ausgebildet, dass einerseits das Eindringen von kontaminierenden Substanzen verhindert und andererseits ein Öffnen durch die komplementäre Betätigungsvorrichtung 40 ermöglicht wird.

Fig. 4a zeigt ebenfalls eine Schnittansicht eines proximalen Endes eines Elements von Gaszuführungseinrichtungen 20, beispielsweise eines proximalen Endes 21b einer Sonde 21, d. h. eines Sondensteckers 22, mit einer Sperreinrichtung 30. Dabei versperrt ein in dem Stecker 22 angeordneter Kugelkörper 33 der Sperreinrichtung 30 eine Gas- und/oder Flüssigkeitsströmung sowohl in als auch entgegen einer Strömungsrichtung S von dem APC-Instrument 11 in Richtung Operationsgebiet. Der Kugelkörper 33 ist in diesem Ausführungsbeispiel mit einem Federelement 33a ausgebildet. Fig. 4b zeigt ein distales Ende eines Elements von Gaszuführungseinrichtungen, z. B. ein distales Ende 23a einer Anschlussleitung 23 zum Verbinden der Sonde 21 mit dem APC-Instrument 11, wobei das Element ausgebildet ist, um mit dem Element gemäß Fig. 4a zusammenzuwirken. Eine zu der Sperreinrichtung 30 komplementäre Betätigungsvorrichtung 40 ist integral mit der Anschlussleitung 23 ausgebildet. Gemäß Fig. 4b weist die Betätigungsvorrichtung 40 und damit die Anschlussleitung 23 ein stößelartiges Ende 45 auf.

Bei Verbindung beider Elemente, der Sonde 21 und der Anschlussleitung 23, drückt die Betätigungsvorrichtung 40 mit dem stößelförmigen Ende 45 den Kugelkörper 33 entgegen einer durch das Federelement 33a bedingten Federkraft in Strömungsrichtung S aus seiner Sperrposition in eine Öffnungsposition. Damit wird der Gasweg für das an dem Operationsgebiet benötigte Arbeitsgas freigegeben. Solange das Arbeitsgas an das Operationsgebiet fließt, wird ein retrograder Fluss von Arbeitsgas, körpereigenen Gasen und/oder körpereigenen Flüssigkeiten verhindert. Bei Entfernung der Betätigungsvorrichtung 40 stellt das Federelement 33a den Kugelkörper 33 in seine Sperrposition zurück, so dass die Sperreinrichtung 30 wieder in und entgegen der Strömungsrichtung S sperrt.

Alternativ ist es möglich, dass Fig. 4b eine Kanüle mit einem stößelförmigen Ende zeigt, die beispielsweise an oder in einer Anschlussleitung explizit vorgesehen ist und entsprechend obiger Beschreibung wirkt.

Wie in den Fig. 3a, 3b und 4a, 4b gezeigt, sind die Elemente der Gaszuführungseinrichtungen 20 durch Stecken miteinander verbindbar. Dazu weisen sowohl die die Sperreinrichtungen 30 aufweisenden Elemente als auch die die Betätigungsvorrichtung 40 aufweisenden Elemente Stoppelemente 13 auf, die die Eindringtiefe des distalen Endes 23a der Anschlussleitungen 23 in das proximale Ende 21b der Sonde 21 bzw. in den Sondenstecker 22 begrenzen. Alternativ kann hier auch eine Schraubverbindung vorgesehen sein, bei der die Elemente jeweils mit Gewinde und Gegengewinde ausgebildet sind.

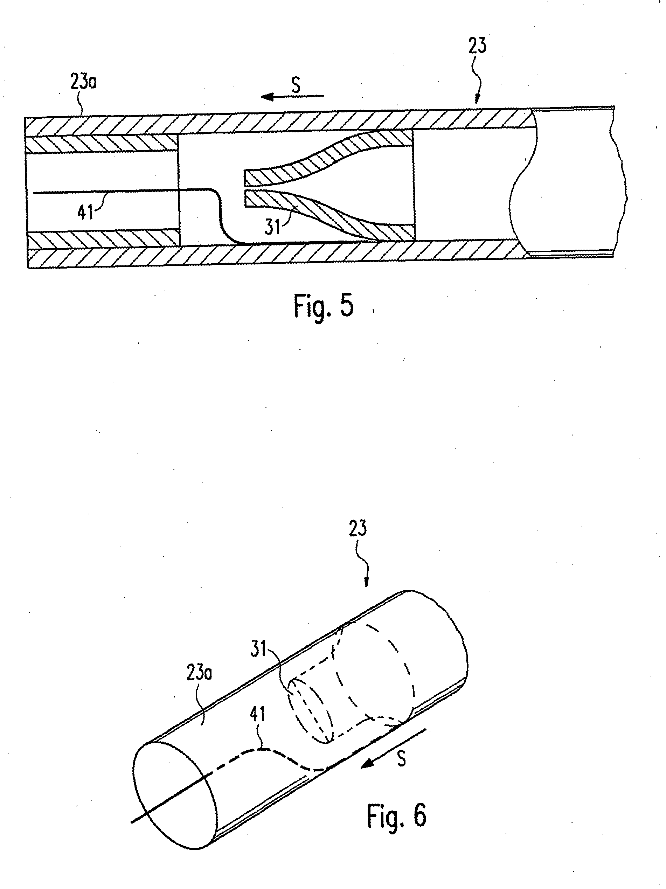

Fig. 5 zeigt eine Schnittansicht eines distalen Endes eines Elements von Gaszuführungseinrichtungen, z. B. eines distalen Endes 23a einer Anschlussleitung 23, mit einer Sperreinrichtung und einer Betätigungsvorrichtung. In der Anschlussleitung 23 ist eine bereits oben beschriebene Lippendichtung 31 vorgesehen. Zusätzlich weist die Anschlussleitung 23 einen Dorn 41 als Betätigungsvorrichtung für ein in Strömungsrichtung S nachfolgendes Element der Gaszuführungseinrichtungen 20 auf. Der Dorn 41 ist vollständig innerhalb der Anschlussleitung 23 ausgebildet, so dass ein die Sperreinrichtung aufweisendes Element der Gaszuführungseinrichtungen derart in das die Betätigungsvorrichtung aufweisende Element einzuführen ist, dass auf diese Art ein Zusammenwirken von Betätigungsvorrichtung und Sperreinrichtung ermöglicht wird.

Fig. 6 zeigt ein ähnliches Ausführungsbeispiel wie das in Fig. 5 dargestellte, allerdings in perspektivischer Ansicht. Das nachfolgende Element, beispielsweise eine Sonde, würde dann bei beiden Ausführungsbeispielen mit einer durch den Dorn 41 öffenbaren Sperreinrichtung, wie etwa eine Membran, ausgebildet sein. Bei Zusammenfügen von Anschlussleitung und Sonde würde die Membran von dem Dorn 41 durchstochen werden, ähnlich wie unter Fig. 3a bzw. 3b beschrieben.

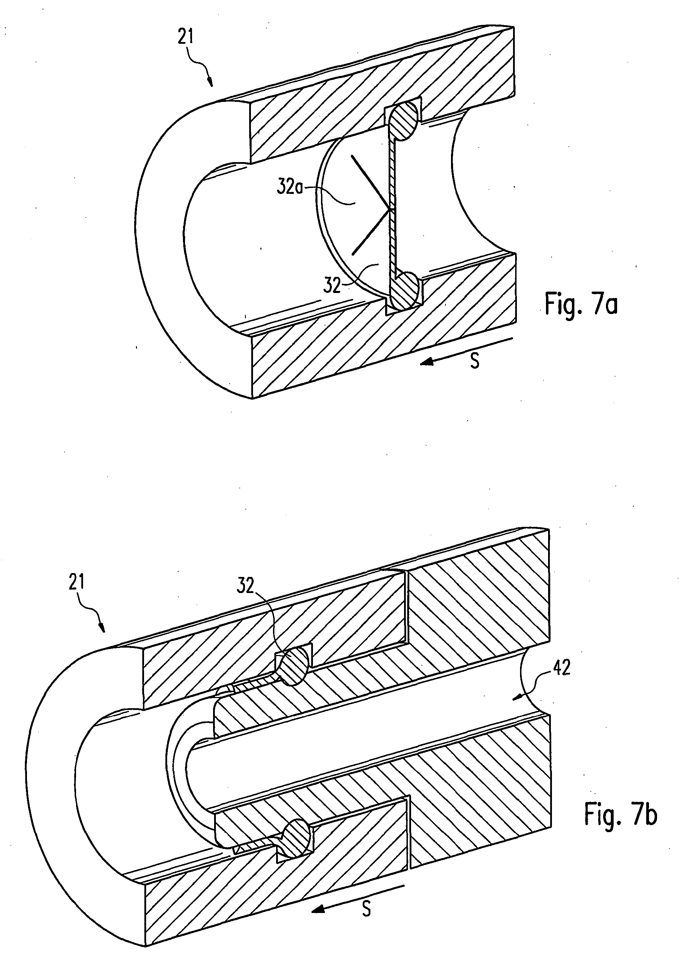

Fig. 7a zeigt eine perspektivische Schnittansicht einer Sperreinrichtung in einem Element von Gaszuführungseinrichtungen, beispielsweise in einer Sonde 21. Die Sperreinrichtung ist hier als elastische Membran 32 ausgebildet, wobei die Membran 32 eine definierte Öffnungsstelle, d. h. eine Soll Öffnungsstelle 32a aufweist. In geschlossenem Zustand ist die Membran 32 für Gase und/oder Flüssigkeiten nahezu undurchlässig.

Fig. 7b zeigt eine perspektivische Ansicht der Sperreinrichtung gemäß Fig. 7a in betätigtem Zustand. Dabei ist eine Kanüle 42 als Betätigungsvorrichtung vorgesehen. Die Kanüle 42 kann beispielsweise an bzw. in einer Anschlussleitung (hier nicht gezeigt) vorgesehen sein. Mit dem Durchbohren der Membran 32 an der dafür vorgesehenen Öffnungsstelle 32a, weichen der Kanüle 42 mindestens Teile der Membran 32 elastisch aus. Bei Entfernung der Kanüle 42 nehmen die Teile der Membran 32 wieder ihre ursprüngliche Position ein, d. h., die Membran 32 ist wieder geschlossen. Wie in Fig. 7b dargestellt, schmiegen sich die ausweichenden Teile der Membran 32 an einer Mantelfläche der Kanüle 42 an, wobei das Arbeitsgas durch die Kanüle 42 strömt.

Die elastische Membran 32 kann auch derart ausgebildet sein, dass das in Strömungsrichtung S fließende Arbeitsgas aufgrund seines Gasdrucks das Öffnen der Membran 32 bewirkt, während ein retrograder Fluss von Arbeitsgas, körpereigenen Gasen und/oder körpereigenen Flüssigkeiten den für das Öffnen der Membran 32 erforderlichen Druck nicht aufbringt. Auf eine explizite Betätigungsvorrichtung, wie sie in Fig. 7b gezeigt ist, kann dann verzichtet werden.

Es sei darauf hingewiesen, dass die Sperreinrichtungen und ggf. komplementäre Betätigungsvorrichtungen in beliebigen Elementen der Gaszuführungseinrichtungen 20 vorgesehen werden können. Insbesondere mehrfach ausgebildete, den Rückfluss kontaminierender Substanzen verhindernde Sperreinrichtungen schützen die Elemente der APC-Einrichtung 10 zuverlässig vor einer Verunreinigung.

### Bezugszeichenliste

- 10: Einrichtung für die Argon Plasma-Koagulation
- 11: Argon-Plasma-Koagulations-Instrument
- 12: Gasquelle
- 13: Stoppelement
- 20: Gaszuführungseinrichtungen
- 21: Sonde
- 21a: Distales Ende
- 21b: Proximales Ende
- 22: Sondenstecker
- 23: Anschlussleitung
- 23a: Distales Ende
- 24: Gasanschlussstück
- 30: Sperreinrichtung
- 31: Lippendichtung
- 32: Membran
- 32a: Definierte Öffnungsstelle
- 33: Kugelkörper
- 33a: Federelement
- 40: Betätigungsvorrichtung
- 41: Dorn
- 42: Kanüle
- 44: Anschrägung
- 45: Stößelförmiges Ende
- S: Strömungsrichtung

## Patentansprüche

1. Einrichtung für die Argon-Plasma-Koagulation,
bei welcher Arbeitsgas mittels Gaszuführungseinrichtungen (20) in einer Strömungsrichtung (S) an ein zu behandelndes Gewebe geführt wird und das Arbeitsgas aus einem distalen Ende der Gaszuführungseinrichtungen (20) ausströmt, wobei
mindestens eine an den Gaszuführungseinrichtungen (20) angeschlossene oder an diese anschließbare Sperreinrichtung (30) vorgesehen ist, zum Sperren einer Gas- und/oder Flüssigkeitsströmung in den Gaszuführungseinrichtungen (20) entgegen der Strömungsrichtung (S)
**dadurch gekennzeichnet, dass**
die Sperreinrichtung (30) durch eine Betätigungsvorrichtung (40) öffenbar ist, wobei die Betätigungsvorrichtung (40) an einem Element der Gaszuführungseinrichtungen (20) angeschlossen oder an dieses anschließbar ist, das in einer Richtung entgegen der Strömungsrichtung (S) und damit vor einem die Sperreinrichtung (30) aufweisenden Element angeordnet ist, so dass ein Öffnen der Sperreinrichtung (30) durch ein Verbinden beider Elemente erfolgt.

2. Einrichtung nach Anspruch 1,
**dadurch gekennzeichnet, dass**
die Sperreinrichtung (30) an einem Rohr, einer schlauchförmige Sonde oder dergleichen chirurgischen Instrument (21) der Gaszuführungseinrichtungen angeschlossen oder an dieses anschließbar ist.

3. Einrichtung nach Anspruch 2,
**dadurch gekennzeichnet, dass**
die Sperreinrichtung (30) an einem Anschlussstück (22) des chirurgischen Instruments (21) der Gaszuführungseinrichtungen angeschlossen oder an dieses anschließbar ist.

4. Einrichtung nach einem der vorhergehenden Ansprüche 2 order 3,
**dadurch gekennzeichnet, dass**
die Sperreinrichtung (30) an Anschlussleitungen (23) zum Anschließen des chirurgischen Instruments (21) an ein Argon-Plasma-Koagulations-Instrument (11) der Einrichtung für die Argon-Plasma-Koagulation (10) angeschlossen oder an diese anschließbar ist.

5. Einrichtung nach Anspruch 4,
**dadurch gekennzeichnet, dass**
die Sperreinrichtung (30) an einem an dem Argon-Plasma-Koagulations-Instrument (11) angeordneten Gasanschlussstück (24) angeschlossen oder an dieses anschließbar ist.

6. Einrichtung nach einem der vorhergehenden Ansprüche,
**dadurch gekennzeichnet, dass**
die Sperreinrichtung (30) als eine mechanisch und/oder elektrisch betätigbare Einrichtung ausgebildet ist.

7. Einrichtung nach einem der vorhergehenden Ansprüche,
**dadurch gekennzeichnet, dass**
die Sperreinrichtung (30) als Sperrventil ausgebildet ist.

8. Einrichtung nach Anspruch 7
**dadurch gekennzeichnet, dass**
das Sperrventil als Lippendichtung (31) ausgebildet ist.

9. Einrichtung nach einem der vorhergehenden Ansprüche,
**dadurch gekennzeichnet, dass**
die Sperreinrichtung (30) als ein durch die Betätigungsvorrichtung (40) irreversibel öffenbares Element ausgebildet ist.

10. Einrichtung nach Anspruch 9,
**dadurch gekennzeichnet, dass**
das irreversibel öffenbare Element als Membran (32) ausgebildet ist.

11. Einrichtung nach einem der vorhergehenden Ansprüche 1-8,
**dadurch gekennzeichnet, dass**
die Sperreinrichtung (30) als ein durch die Betätigungsvorrichtung (40) mehrfach öffenbares Element ausgebildet ist.

12. Einrichtung nach Anspruch 11,
**dadurch gekennzeichnet, dass**
das mehrfach öffenbare Element als Membran (32) mit einer definierten Öffnungsstelle (32a) ausgebildet ist.

13. Einrichtung nach Anspruch 11,
**dadurch gekennzeichnet, dass**
das mehrfach öffenbare Element als Sperreinrichtung (30) mit einem die Gas- und/oder Flüssigkeitsströmung versperrenden und von der Betätigungsvorrichtung (40) verschiebbaren Körper (33) ausgebildet ist.

14. Einrichtung nach einem der vorhergehenden Ansprüche,
**dadurch gekennzeichnet, dass**
die Betätigungsvorrichtung (40) als Dorn (41), Kanüle (42) oder Stößel ausgebildet ist.

15. Einrichtung nach einem der vorhergehenden Ansprüche,
**dadurch gekennzeichnet, dass**
die Sperreinrichtung (30) einen Garantieverschluss umfasst.

## Claims

1. Device for argon plasma coagulation,
in which working gas is conducted, by means of gas supply devices (20), in a flow direction (S) to tissue to be treated and the working gas flows out of the distal end of the gas supply devices (20),
there being provided at least one blocking device (30), which is connected or connectible to the gas supply devices (20), for blocking a flow of gas and/or liquid into the gas supply devices (20) in a direction opposite to the flow direction (S),
**characterized in that**
the blocking device (30) is openable by an actuating device (40), the actuating device (40) being connected or connectible to an element of the gas supply devices (20) that is oriented opposite to the flow direction (S) and therefore upstream of an element having the blocking device (30), so that opening of the blocking device (30) is effected by connection of the two elements.

2. Device according to claim 1,
**characterized in that**
the blocking device (30) is connected or connectible to a tube, a hose-like probe or similar surgical instrument (21) of the gas supply devices.

3. Device according to claim 2,
**characterized in that**
the blocking device (30) is connected or connectible to a connection piece (22) of the surgical instrument (21) of the gas supply devices.

4. Device according to either one of the preceding claims 2 and 3,
**characterized in that**
the blocking device (30) is connected or connectible to connection lines (23) for connection of the surgical instrument (21) to an argon plasma coagulation instrument (11) of the device for argon plasma coagulation (10).

5. Device according to claim 4,
**characterized in that**
the blocking device (30) is connected or connectible to a gas connection piece (24) arranged on the argon plasma coagulation instrument (11).

6. Device according to any one of the preceding claims,
**characterized in that**
the blocking device (30) is in the form of a mechanically and/or electrically operable device.

7. Device according to any one of the preceding claims,
**characterized in that**
the blocking device (30) is in the form of a blocking valve.

8. Device according to claim 7,
**characterized in that**
the blocking valve is in the form of a lip seal (31).

9. Device according to any one of the preceding claims,
**characterized in that**
the blocking device (30) is in the form of an element irreversibly openable by the actuating device (40).

10. Device according to claim 9,
**characterized in that**
the irreversibly openable element is in the form of a membrane (32).

11. Device according to any one of the preceding claims 1 to 8,
**characterized in that**
the blocking device (30) is in the form of an element that is multiply openable by the actuating device (40).

12. Device according to claim 11,
**characterized in that**
the multiply openable element is in the form of a membrane (32) having a defined opening location (32a).

13. Device according to claim 11,
**characterized in that**
the multiply openable element is in the form of a blocking device (30) having a body (33) that blocks the flow of gas and/or liquid and is displaceable by the actuating device (40).

14. Device according to any one of the preceding claims,
**characterized in that**
the actuating device (40) is in the form of a spike (41), cannula (42) or plunger.

15. Device according to any one of the preceding claims,
**characterized in that**
the blocking device (30) comprises a tamper-evident closure.

## Revendications

1. Dispositif pour la coagulation du plasma d'argon,
sur lequel du gaz de travail est guidé au moyen de dispositifs d'arrivée de gaz (20) dans un sens d'écoulement (S) sur un tissu à traiter et le gaz de travail sort d'une extrémité distale des dispositifs d'arrivée de gaz (20),
au moins un dispositif de blocage (30) raccordé aux dispositifs d'arrivée de gaz (20) ou pouvant être raccordé à ces dispositifs étant prévu, pour le blocage d'un écoulement de gaz et/ou de liquide dans les dispositifs d'arrivée de gaz (20) dans le sens contraire au sens d'écoulement (S),
**caractérisé en ce que**
le dispositif de blocage (30) peut être ouvert par un dispositif d'actionnement (40), le dispositif d'actionnement (40) étant raccordé à un élément des dispositifs d'arrivée de gaz (20) ou pouvant être raccordé à cet élément, lequel est disposé dans une direction contraire au sens d'écoulement (S) et donc en amont d'un élément présentant le dispositif de blocage (30), de sorte qu'une ouverture du dispositif de blocage (30) s'effectue par une liaison des deux éléments.

2. Dispositif selon la revendication 1,
**caractérisé en ce que**
le dispositif de blocage (30) est raccordé à un tuyau, une sonde en forme de flexible ou un instrument (21) chirurgical similaire des dispositifs d'arrivée de gaz ou peut être raccordé à celui-ci.

3. Dispositif selon la revendication 2,
**caractérisé en ce que**
le dispositif de blocage (30) est raccordé à un raccord (22) de l'instrument (21) chirurgical des dispositifs d'arrivée de gaz ou peut être raccordé à celui-ci.

4. Dispositif selon l'une quelconque des revendications 2 ou 3 précédentes,
**caractérisé en ce que**
le dispositif de blocage (30) est raccordé à des conduites de branchement (23) pour le raccordement de l'instrument (21) chirurgical à un instrument de coagulation du plasma d'argon (11) du dispositif pour la coagulation du plasma d'argon (10) ou peut être raccordé à celui-ci.

5. Dispositif selon la revendication 4,
**caractérisé en ce que**
le dispositif de blocage (30) est raccordé à un raccord de gaz (24) disposé sur l'instrument de coagulation du plasma d'argon (11) ou peut être raccordé à celui-ci.

6. Dispositif selon l'une quelconque des revendications précédentes,
**caractérisé en ce que**
le dispositif de blocage (30) est réalisé sous forme d'un dispositif pouvant être actionné mécaniquement et/ou électriquement.

7. Dispositif selon l'une quelconque des revendications précédentes,
**caractérisé en ce que**
le dispositif de blocage (30) est réalisé sous forme de clapet antiretour.

8. Dispositif selon la revendication 7,
**caractérisé en ce que**
le clapet antiretour est réalisé sous forme de joint à lèvre (31).

9. Dispositif selon l'une quelconque des revendications précédentes,
**caractérisé en ce que**
le dispositif de blocage (30) est réalisé sous forme d'un élément pouvant être ouvert de façon irréversible par le dispositif d'actionnement (40).

10. Dispositif selon la revendication 9,
**caractérisé en ce que**
l'élément pouvant être ouvert de façon irréversible est réalisé sous forme de membrane (32).

11. Dispositif selon l'une quelconque des revendications 1 à 8 précédentes,
**caractérisé en ce que**
le dispositif de blocage (30) est réalisé sous forme d'un élément pouvant être ouvert plusieurs fois par le dispositif d'actionnement (40).

12. Dispositif selon la revendication 11,
**caractérisé en ce que**
l'élément pouvant être ouvert plusieurs fois est réalisé sous forme de membrane (32) avec un point d'ouverture (32a) défini.

13. Dispositif selon la revendication 11,
**caractérisé en ce que**
l'élément pouvant être ouvert plusieurs fois est réalisé sous forme de dispositif de blocage (30) avec un corps (33) bloquant l'écoulement de gaz et/ou de liquide et pouvant être déplacé par le dispositif d'actionnement (40).

14. Dispositif selon l'une quelconque des revendications précédentes,
**caractérisé en ce que**
le dispositif d'actionnement (40) est conçu sous forme de mandrin (41), de canule (42) ou de coulisseau.

15. Dispositif selon l'une quelconque des revendications précédentes,
**caractérisé en ce que**
le dispositif de blocage (30) présente une fermeture de garantie.
